# EUROPEAN PATENT APPLICATION

(11) **EP 2 308 394 A1**
(43) Date of publication of application: **13.04.2011**
(21) Application number: 10251750.5
(22) Date of filing: 06.10.2010
(51) Int. Cl.: A61B 17/122, A61B 17/128, A61B 17/02, A61B 17/00

(54) **Spring jaw retraction device**

(30) Priority: 07.10.2009 US 249312 P; 01.10.2010 US 895956
(71) Applicant: Tyco Healthcare Group LP, New Haven, CT 06511 (US)
(72) Inventor: Okoniewski, Gregory, North Haven, CT 06473 (US)
(74) Representative: Soames, Candida Jane

(57) **Abstract**

A retraction assembly is provided for retracting body tissue away from an operative site. The retraction device generally includes a first jaw and a second jaw connected by a biasing member. The biasing member urges distal ends of the first and second jaws together to grasp the body tissue. An anchoring assembly is connected to the retraction device for reposition the grasped target body tissue away from the operative site. The anchoring assembly includes a surgical needle and a length of suture material attached to the retraction device. There is also provided an applicator instrument having a hollow outer tube for receipt of the retraction device and to position and advance the retraction device over the body tissue. A pusher is provided and is movable within the hollow outer tube, and engageable with the retraction device, to force the retraction device out of the hollow outer tube and about the body tissue.

## Description

### CROSS-REFERENCE TO RELATED APPLICATIONS

The present application claims the benefit of and priority to U.S. Provisional Patent Application Serial No. 61/249,312, filed October 7, 2009, the disclosure of which is herein incorporated by reference in its entirety.

### BACKGROUND

### 1. Technical field

The present disclosure relates to a spring jaw retraction device. More particularly, the present disclosure relates to a biased retraction device and applicator instrument for engaging and retracting an organ, or part thereof, away from an operative site within a body cavity.

### 2. Background Of Related Art

Many surgical procedures are currently performed in a minimally invasive manner so as to limit the amount of trauma to the patient and promote more rapid healing. These minimally invasive procedures generally include forming one or more incisions through the body wall of a patient and inserting the operative surgical instruments through the incisions. In some instances, access ports are inserted through the incisions and are provided to receive surgical instruments therethrough. In some surgical procedures, it is often necessary to move or retract a portion of the body, such as a body organ, away from the operative site to facilitate performing the surgery. This is often accomplished by inserting an elongate retraction instrument through the incision or access port and utilizing movable jaw structure located at a distal end of the surgical instrument to grasp the body organ and move or retract the body organ away from the operative site.

In these methods of retracting body organs, the elongate retraction instrument remains positioned through the body wall and occupies the incision or access port during the entire surgical procedure. In complex surgical procedures, multiple incisions or access ports are required to accommodate the multiple surgical instruments used during the surgical procedures. Each incision or access port utilized in the surgical procedure contributes to the degree of trauma and rate of healing of the patient.

Therefore, it is desirable to provide retraction device assembly including an applicator instrument and a deployable retraction device which can be inserted through a surgical incision or access port to engage and retract a body organ while allowing for removal of the applicator instrument from the incision or access port during the surgical procedure so as to limit the number of incisions or access ports required.

### SUMMARY

There is disclosed a surgical retraction assembly for retracting a body organ or tissue against an abdominal wall. The retraction assembly includes a retraction device generally including first and second jaw members. The retraction assembly additionally includes a biasing member connecting the first jaw member to the second jaw member. The biasing member urges the distal ends of the first and second jaw members toward one another. The retraction assembly further includes a anchoring assembly connected to the retraction device.

Relative movement between the actuator and the outer tubes results in the retraction device translating through the applicator instrument.

In a particular embodiment, the first jaw, the second jaw and the biasing member are integrally formed. In a specific embodiment the biasing member is a spring.

The applicator instrument may include a hollow outer tube having an open distal end for receipt of the retraction device. The applicator instrument also has a pusher, having a distal pusher face, moveable within the outer tube such that distal movement of the pusher through the outer tube causes the pusher face to move the retraction device out of the open distal end of the outer tube.

The retraction device additionally includes an anchoring assembly for repositioning body tissue positioned between the first and second jaw members. The anchoring assembly generally includes a surgical needle having a tissue penetrating tip and a length of suture material connecting the surgical needle to the first jaw.

There is further disclosed a method of capturing and retracting body tissue. The method includes providing retraction assembly having an applicator instrument containing a tube and a pusher positioned within the tube, and a retraction device having first and second jaw members. Retraction assembly further contains a biasing member operably connected to the first and second jaw members urging distal ends of the first and second jaws towards a first condition, and an anchoring assembly connected to the retraction device for repositioning body tissue positioned between the first and second jaw members.

The retraction device is positioned in a second condition within the applicator instrument and the entire retraction assembly is placed into a patient through an opening in their abdominal wall. The retraction device is disengaged from the applicator instrument by repositioning the pusher relative to the tube such that the retraction device translates distally through an open distal end of the tube applicator and then the first and second jaw members close around a portion of body tissue. The applicator and pusher are then withdrawn. After which the anchor assembly is manipulated to reposition the portion of body tissue.

The anchor assembly may include a surgical needle attached to a length of suture material.

Alternatively, the anchor assembly may include a length of suture material extending proximally through a lumen in the pusher and tube of the applicator instrument. After the retraction device captures body tissue, the applicator instrument is withdrawn relative to the length of suture, leaving one end of the length of suture accessible while the other end is attached to the retraction device.
The invention may be described by reference to the following numbered paragraphs:-
1. A retraction assembly according to the present invention comprising: an applicator instrument comprising: a tube; and a pusher positioned within the tube; a retraction device, the retraction device having first jaw and second jaw members; a biasing member operably connected to the first and second jaw members, the biasing member urging distal ends of the first and second jaws towards a first condition; and an anchoring assembly connected to the retraction device; for use in a method of capturing and retracting body tissue, which method comprises the steps of: providing a retraction assembly according to the present invention positioning the retraction device, in a second condition within the applicator instrument; inserting the retraction assembly into a patient through an abdominal wall; repositioning the pusher relative to the tube such that the retraction device translates distally through a open distal end of the tube; closing the first and second jaw members around a portion of body tissue; and manipulating the anchor assembly to reposition the portion of body tissue.
2. A retraction assembly according to paragraph 1, wherein the anchor assembly includes a surgical needle attached to a length of suture material.

### DESCRIPTION OF THE DRAWINGS

Embodiments of the presently disclosed spring jaw retraction device is disclosed herein with reference to the drawings, wherein:
FIG. 1 is a perspective view of a spring jaw retraction device for engaging and retracting bodily tissue;
FIG. 2 is a perspective view of an applicator instrument for use in inserting the spring jaw retraction device of FIG. 1 into the body of the patient;
FIG. 3 is a side view, partially shown in cross-section, of the applicator instrument of FIG. 2 and spring jaw retraction device of FIG. 1;
FIG. 4 is a side view, partially shown in cross-section, of the applicator instrument being positioned adjacent a body tissue;
FIG. 5 is a side view, partially shown in cross-section, illustrating the spring jaw retraction device being positioned over the body tissue;
FIG. 6 is a side view, partially shown in cross-section, illustrating a pusher of the applicator instrument applying the spring jaw retraction device to the body tissue;
FIG. 7 is a side view, partially shown in section, illustrating the spring jaw retraction device fully ejected from the applicator instrument and fully applied to the body organ; and
FIG. 8 is a side view of an alternate embodiment of the present disclosure illustrating the spring jaw retraction device being positioned over the body tissue.

### DETAILED DESCRIPTION OF EMBODIMENTS

An embodiment of the presently disclosed spring jaw retraction device and applicator instrument will now be described in detail with reference to the drawings wherein like numerals designate identical or corresponding elements in each of the several views. As is common in the art, the term "proximal" refers to that part or component closer to the user or operator, i.e. surgeon or physician, while the term "distal" refers to that part or component further away from the user.

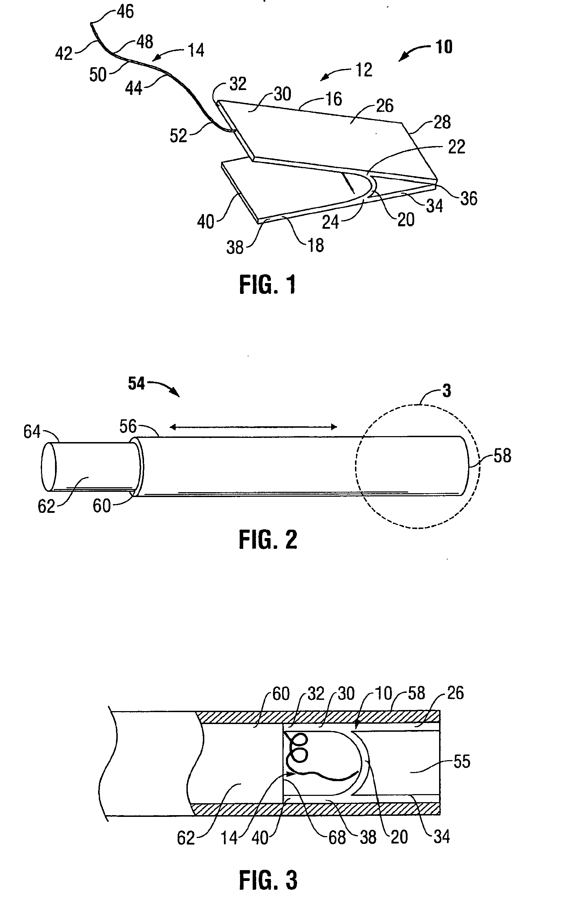

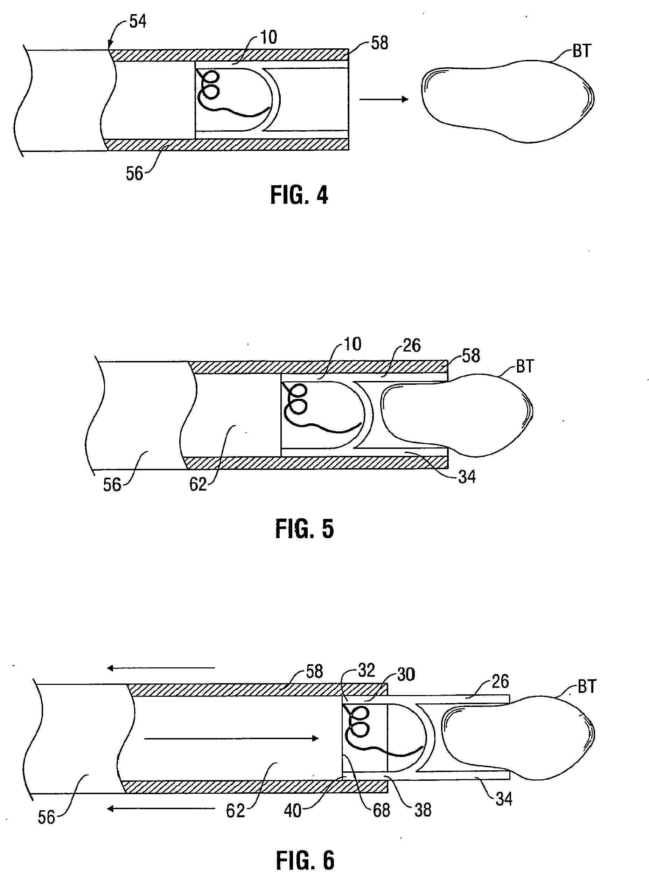

Referring initially to FIG. 1, there is disclosed a spring biased jaws retraction device or retraction device 10. Retraction device 10 is provided to retract and secure body tissue BT (FIG. 4), or part thereof, away from an operative site within a body cavity and generally includes a jaw assembly 12 for engaging the body tissue BT and an anchoring assembly 14 for holding the retracted tissue away from the operative site. Jaw assembly 12 includes a first jaw 16 and a second jaw 18 interconnected by a biasing member or biasing spring 20. A first end 22 of biasing spring 20 is connected to first jaw 16 while a second end 24 of biasing spring 20 is connected to second jaw 18. As shown, in this specific embodiment, biasing spring 20 is relatively flat and has a generally C- shape facing proximally. Alternatively, biasing spring 20 may be formed from a variety of types of biasing members such as, for example, coil springs, leaf springs, living hinge, etc. Additionally, in the disclosed embodiment, first and second jaws 16 and 18, respectively, have generally rectangular shapes.

Biasing spring 20 may be formed integrally with first and second jaws 16 and 18 or may be provided as a separate element. First jaw 16, second jaw 18 and biasing spring 20 may be formed from a variety of materials such as, for example, plastics, metallic materials, etc.

First jaw 16 includes a first clamp jaw 26 extending distally from first end 22 of biasing spring 20 to a distal end 28 of first jaw 16. A first clamp leg 30 of first jaw 16 extends proximally from first end 22 of biasing spring 20 to a proximal end 32 of first jaw 16. Likewise, second jaw 18 includes a second clamp jaw 34 extending distally from second end 24 of biasing spring 20 to a distal end 36 of second jaw 18 and a second clamp leg 38 extending proximally from second end 24 of biasing spring 20 to a proximal end 40 of second jaw 18. In the unconstrained condition, biasing spring 20 biases distal ends 28 and 36 of first and second jaws 16 and 18, respectively, together to allow first and second clamp jaws 26 and 34 to engage and hold body tissue BT.

As noted herein above, retraction device 10 includes an anchoring assembly 14 to facilitate securing jaw assembly 12, and captured body tissue BT, against an abdominal wall and away from an operative site. Anchoring assembly 14 includes a surgical needle 42 having a length of suture material 44 affixed to surgical needle 42. Surgical needle 42 includes a tissue penetrating tip 46 for piercing an abdominal wall. One end 48 of surgical needle 42 is connected to a first end 50 of length of suture material 44. A second end 52 of length of suture material 44 is connected to proximal end 32 of first jaw 16.

Tissue penetrating tip 46 is provided to pierce an abdominal wall allowing surgical needle 42 to pass through the abdominal wall. Drawing surgical needle 42 through the abdominal wall also draws length of suture material 44 through the abdominal wall such that jaw assembly 12, having body tissue BT engaged by jaw assembly 12, is drawn toward or retracted against the abdominal wall.

Referring now to FIGS. 2 and 3, there is also disclosed an applicator instrument 54 for retaining retraction device 10 during insertion through an abdominal wall and deploying retraction device 10 about body tissue BT. Applicator instrument 54 generally includes an outer member or hollow outer tube 56 and an inner member, actuator or pusher 62, where inner member 62 is positionable axially with respect to outer member 56. Outer member 56 has a passageway 55 for slidably receiving retraction device 10. More specifically a hollow outer tube 56 has an open distal end 58, for receipt of retraction device 10, and an open proximal end 60 that defines passageway 55. Applicator instrument 54 also includes a pusher 62 movable within outer tube 56. Pusher 62 has a proximal portion 64 extending proximally out of proximal end 60 of outer tube 56 and a distal portion 66 having a pusher face 68. Pusher face 68 engages and moves retraction device 10 out of outer tube 56 in order to grasp and retract body tissue BT.

Referring now to FIGS. 3-7, and initially with regard to FIG. 3 the use of retraction device 10 and applicator instrument 54 will now be described. As shown, retraction device 10 is initially positioned within outer tube 56 and generally near distal end 58. In order to accomplish this, first and second clamp legs 30 and 38 are forced together against the bias of spring 20 to allow retraction device 10 to be inserted into outer tube 56. As noted hereinabove, moving first and second clamp legs 30 and 38 together forces first and second clamp jaws 26 and 34 into an open or spaced apart position capable of engaging body tissue BT. As further shown, anchoring assembly 14 is also contained within outer tube 56.

In the initial position, pusher face 68 of pusher 62 abuts proximal ends 32 and 40 of first and second jaws 16 and 18, respectively.

Referring now to FIGS. 4 and 5, after insertion through an opening or incision in the abdominal wall, distal end 58 of outer tube 56 is advanced toward body tissue BT to be retracted. With specific reference to FIG. 5, distal end 58 of outer tube 56 is advanced over a portion of the body tissue BT such that first and second clamp jaws 26 and 34 surround a portion of the body tissue BT.

Referring to FIG. 6, once first and second clamp jaws 26 and 34 have been properly positioned about the body tissue BT, pusher 62 is advanced distally through outer tube 56 causing pusher face 68 to engage proximal ends 32 and 40 of first and second jaws 16 and 18 and thus drive first and second clamp legs 30 and 38 distally within outer tube 56. In an alternative method, outer tube 56 is retracted proximally over pusher 62 so as to avoid any additional distal pressure to the body tissue BT by first and second clamp jaws 26 and 34. In either scenario, relative movement between the outer tube 56 and pusher 62 urges retraction device distally through open distal end 58.

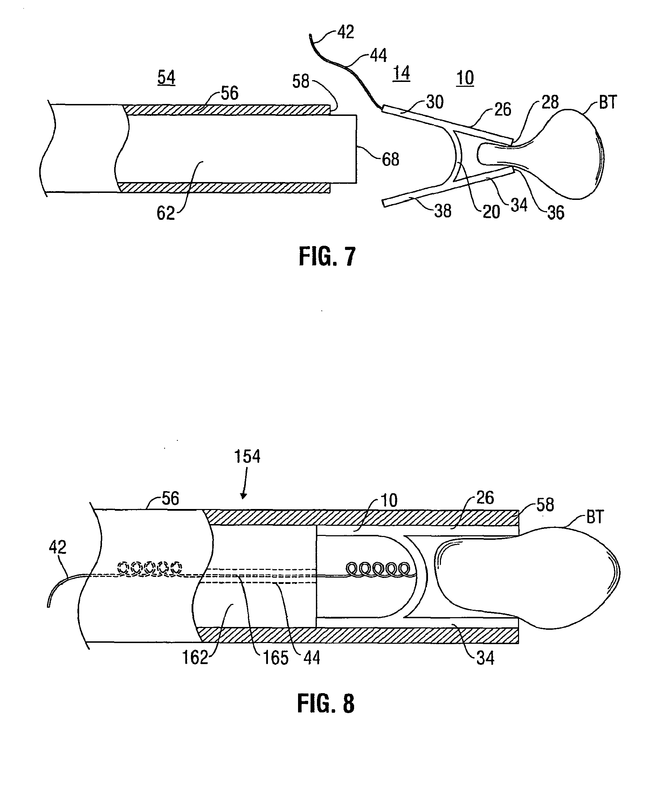

Referring now to FIG. 7, as pusher 62 is advanced fully through outer tube 56, pusher face 68 forces retraction device 10 completely out of distal end 58 of outer tube 56. Upon exiting outer tube 56, first and second clamp jaws 26 and 34 are forced together to a closed position about the body tissue BT by the bias of spring 20. Thereafter, applicator instrument 54 is removed from the operative site.

Anchoring assembly 14 is then used to retract and secure the body tissue BT away from the operative site. Specifically, surgical needle 42 is forced through the abdominal wall to draw length of suture material 44, and thus the body tissue BT engaged by first and second jaws 16 and 18, away from the operative site. Suture material 44 and surgical needle 42 may be composed of a bio-absorbable material. Alternatively, surgical needle 42 may be grasped by separate surgical instrument and manipulated through an access port inserted through the abdominal wall to draw and remove the target body tissue BT out of patient's body.

FIG. 8 shows an alternative embodiment of the applicator instrument shown generally as 154. Applicator instrument 154 includes outer tube 56 and retraction device 10 as previously shown and described. Additionally, applicator instrument 154 includes a pusher 162 that is similar to pusher 62 that was previously shown and described and further includes a lumen 163 extending from the proximal end to the distal end of pusher 162. The length of suture material 44 attached to the anchor assembly 14 (FIG. 1) extends proximally through the lumen 163 in the pusher 162 and exits through the outer tube 56 of the applicator instrument 154. After the retraction device 10 captures body tissue BT, the applicator instrument 154 is withdrawn relative to the length of suture 44, leaving one end of the length of suture 44 accessible while the other end is attached to the retraction device 10. Suture 44 may include a needle 42 attached at one end.

It will be understood that various modifications may be made to the embodiments disclosed herein. For example, the disclosed jaws may have configurations other than rectangular, such as, for example, oval, tubular, etc. Further, the disclosed spring may be a coil spring, leaf spring, etc. Additionally, the anchoring assembly may include other types of fastening devices, such as, for example, staples, clips, barbed tags, etc. Therefore, the above description should not be construed as limiting, but merely as exemplifications of particular embodiments. Those skilled in the art will envision other modifications within the scope and spirit of the claims appended hereto.

## Claims

1. A surgical retraction assembly comprising:
an applicator instrument having a hollow outer tube and a actuator;
a retraction device having first and second jaw members, wherein the retraction device is selectively positionable within the applicator instrument;
a biasing member operably connected to the first and second jaw members, the biasing member urging distal ends of the first and second jaws toward one another;
an anchoring assembly connected to the retraction device.

2. The retraction device as recited in claim 1, wherein relative movement between the actuator and the outer tube translates the retraction device through the applicator instrument.

3. The retraction device as recited in claim 1 or claim 2, wherein the first jaw, second jaw and biasing member are integrally formed.

4. The retraction device as recited in any of claims 1 to 3, wherein the biasing member is a spring.

5. The surgical retraction assembly as recited in any preceding claim, wherein the hollow outer tube has an open distal end for receipt of the retraction device.

6. The surgical retraction assembly as recited in claim 5, wherein the actuator includes a distal pusher face, moveable within the hollow outer tube to eject the retraction device from the hollow outer tube.

7. The surgical retraction assembly as recited in any preceding claim, wherein the anchoring assembly includes a surgical needle connected to a length of suture material connecting the surgical needle to one of the jaw members.

8. The surgical retraction assembly as recited in any preceding claim, wherein the anchor assembly includes a length of suture material extending proximally through a lumen in the actuator.

9. A retraction assembly according to any preceding claim comprising:
an applicator instrument comprising:
a tube; and
a pusher positioned within the tube;
a retraction device, the retraction device having first jaw and second jaw members;
a biasing member operably connected to the first and second jaw members, the biasing member urging distal ends of the first and second jaws towards a first condition; and
an anchoring assembly connected to the retraction device; for use in a method of capturing and retracting body tissue, which method comprises the steps of:
providing a retraction assembly according to any preceding claim positioning the retraction device, in a second condition within the applicator instrument; inserting the retraction assembly into a patient through an abdominal wall;
repositioning the pusher relative to the tube such that the retraction device translates distally through a open distal end of the tube;
closing the first and second jaw members around a portion of body tissue; and
manipulating the anchor assembly to reposition the portion of body tissue.

10. A retraction assembly according to claim 9, wherein the anchor assembly includes a surgical needle attached to a length of suture material.
